Europäisches Patentamt

European Patent Office

Office européen des brevets

(11) Publication number: **0 321 842**
**A1**

## EUROPEAN PATENT APPLICATION

(21) Application number: 88120878.9

(22) Date of filing: 14.12.88

(51) Int. Cl.⁴: **C12N 15/00 , C12N 5/00 ,
A61K 37/02 , C12P 21/02**

(30) Priority: 22.12.87 EP 87119080
20.01.88 EP 88100814

(43) Date of publication of application:
28.06.89 Bulletin 89/26

(84) Designated Contracting States:
AT BE CH DE ES FR GB GR IT LI LU NL SE

(71) Applicant: **Kishimoto, Tadamitsu, Prof.
Division of Cellular Immunology Institute for
Molecular and Cellular Biology University
Osaka
1-3, Yamadaoka Suita Osaka 565(JP)**

(72) Inventor: **Kishimoto, Tadamitsu, Prof. Dr.
3-5-31, Nakano
Tondabayashi Osaka 584(JP)**
Inventor: **Suemura, Masaki, Dr.
9-5, Himemuro
Ikeda Osaka 563(JP)**
Inventor: **Kikutani, Hitoshi, Dr.
2-17-B504, Senriyama-Higashi**

**Suita Osaka 565(JP)**
Inventor: **Barsumian, Edward L., Dr.
3-11-5-503, Senba-Nishi
Mino Osaka 562(JP)**
Inventor: **Schneider, Franz-Josef, Dr.
Fasholdgasse 8/5
A-1130 Vienna(AT)**
Inventor: **Schwendenwein, Renate, Dr.
Braunspergengasse 12/2/16
A-1100 Vienna(AT)**
Inventor: **Sommergruber, Wolfgang, Dr.
Stoesslgasse 6/5
A-1130 Vienna(AT)**
Inventor: **Swetly, Peter, Dr.
Hietzinger Hauptstrasse 40B
A-1130 Vienna(AT)**

(74) Representative: **Laudien, Dieter, Dr. et al
Boehringer Ingelheim Zentrale GmbH ZA
Patente Postfach 200
D-6507 Ingelheim am Rhein(DE)**

(54) Soluble recombinant Fc-Epsilon-receptor, the preparation and the use thereof.

(57) The present invention describes the expression of highly bioactive water-soluble FcεR in cells derived from multicellular organismns and the use thereof.

EP 0 321 842 A1

## Soluble recombinant Fcε-receptor, the preparation and the use thereof

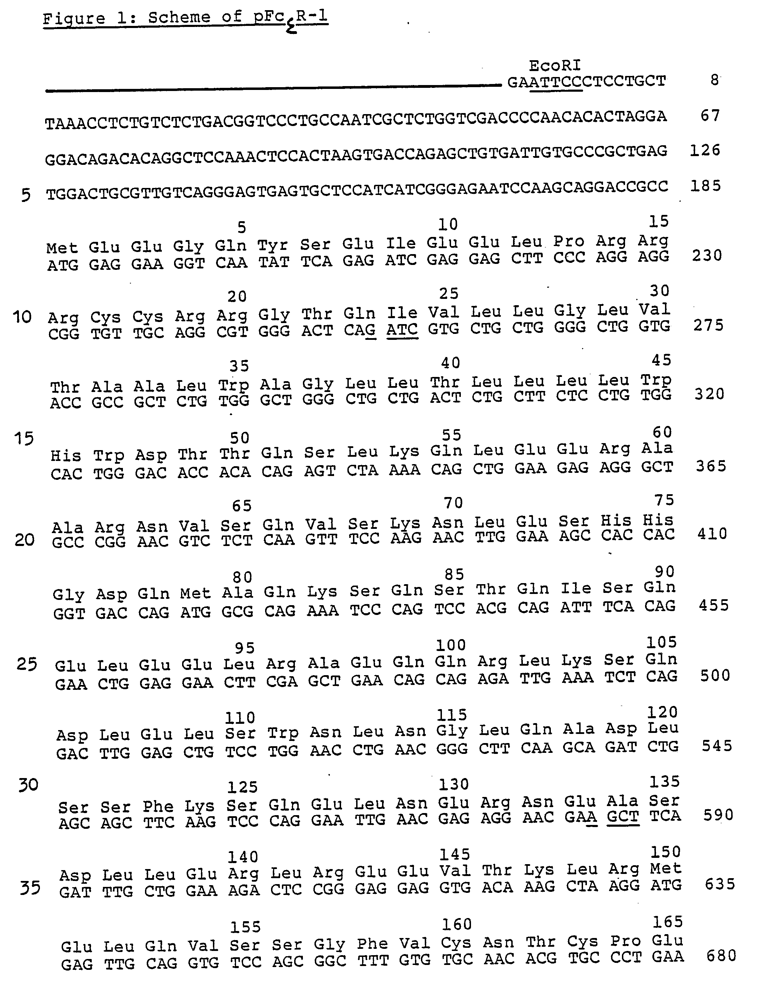

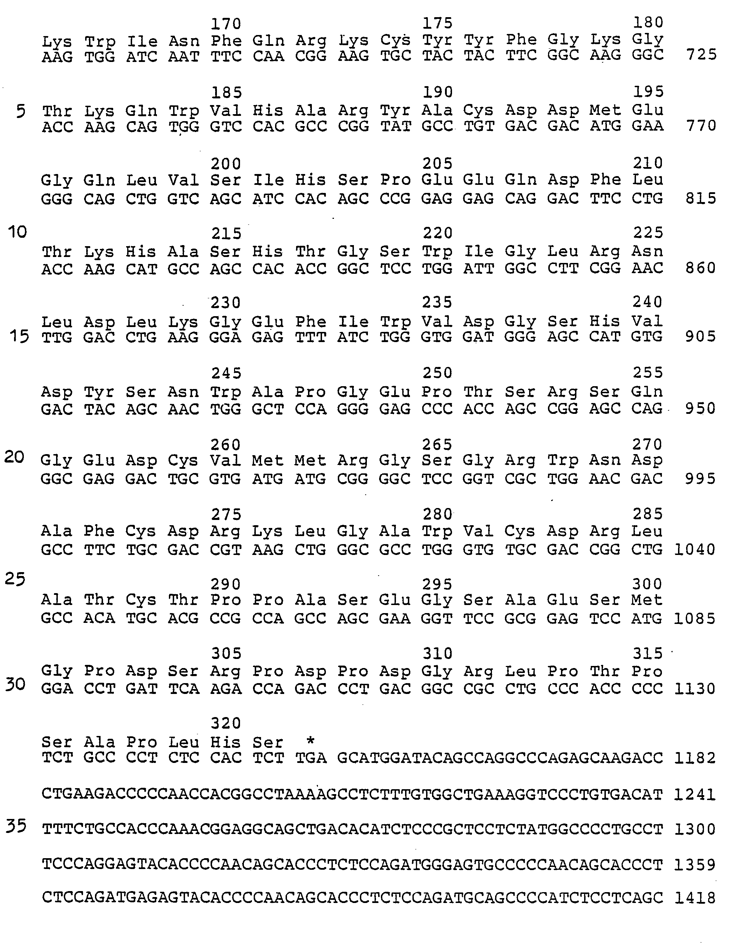

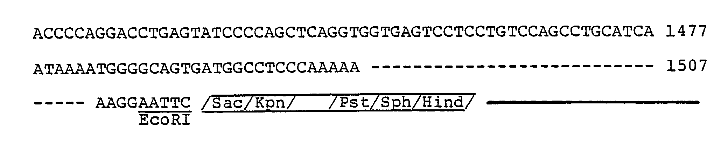

The molecular structure of Human Lymphocyte Receptor for Immunoglobulin (FcεR) as well as its cloning and expression was recently described in the literature by Tadamitsu Kishimoto et al. (see Cell 47, 657-665 (1986)) and by Guy Delespesse et al. (see The Embo Journal 6, 109-114 (1987)). The molecular structure of FcεR (see Figure 1) indicates that it is oriented on the cell membrane with its N-terminus inside the cell and its carboxy terminus exposed outside the cell.

Furthermore, the residue at position 150 of FcεR is a potential cleavage site for trypsin-like proteases, which explains the presence of soluble components of FcεR in culture supernatants of B cells and certain B lymphoblastoid cell lines. This may be the reason for the presence of water-soluble FcεR or a portion of it associated or complexed to IgE in the serum of normal and atopic individuals, with significantly higher levels in the latter group.

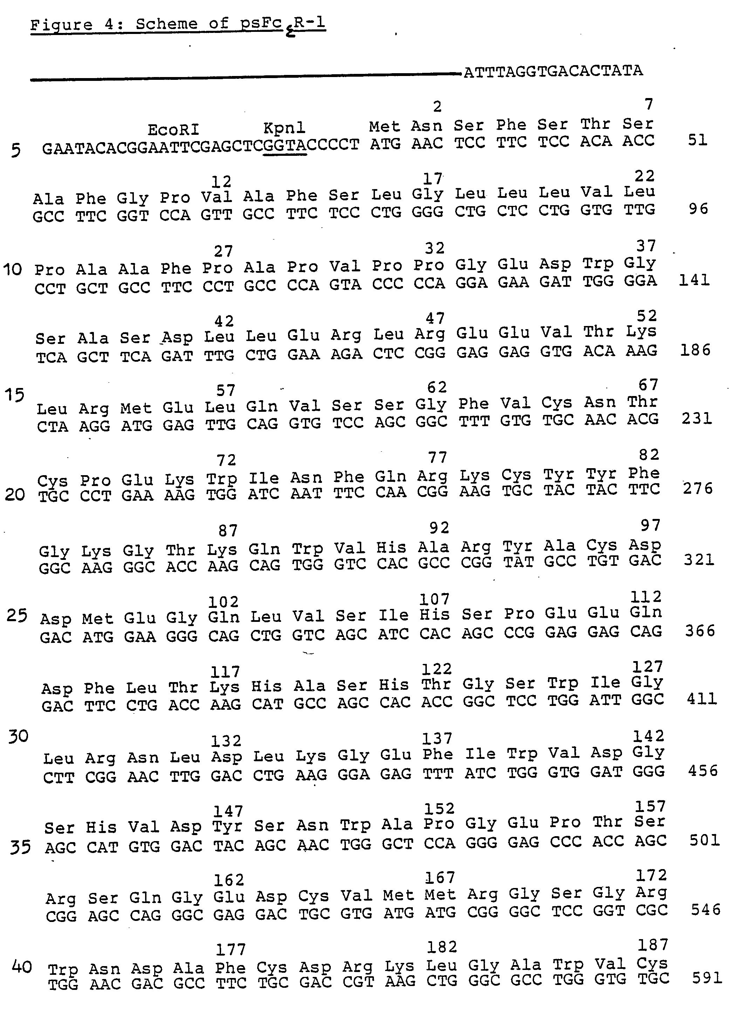

Moreover, in the not yet published European Patent Application Nr. 87111392.4 are described a cDNA-sequence, wherein at least a part of the cDNA sequence coding for the amino acids 1 to 148 is replaced by a suitable cDNA fragment coding for an eucaryotic signal sequence. Thus, in the described plasmid psFcεR-1 (see Figure 4) the coding sequence for the amino acids 1 to 133 is replaced by the BSF-2 signal sequence as follows:

The plasmid LE392 (see Cell 47, 657-665 (1986)) deposited under the terms of the Budapest Treaty under FERM BP-1116 on August 01, 1986 was digested with HindIII, whereby a 1.0 kbp HindIII-fragment was obtained containing the coding sequence for the amino acids 134 to 321 of the full-length FcεR cDNA. The recessed 3'-ends of this fragment were then filled in with the Klenow fragment of DNA polymerase and the DNA subsequently digested with PstI. The obtained fragment was then cloned in a suitable vector, preferably with a BamHI-PstI digested pBSF2-L8, whereby this vector is conveniently prepared as follows:

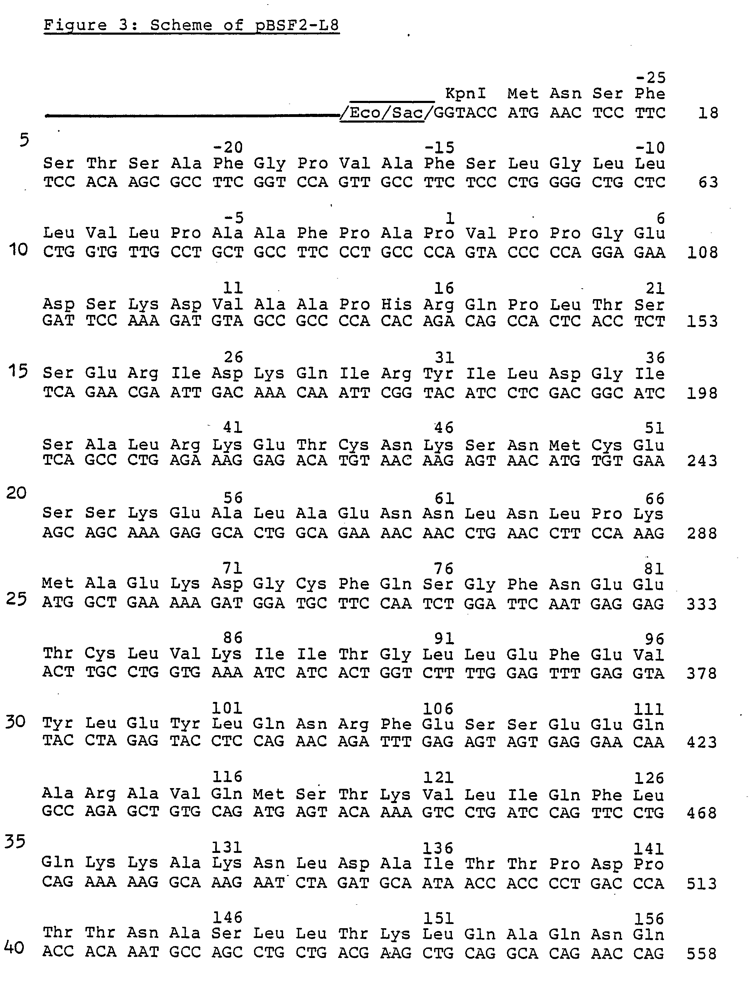

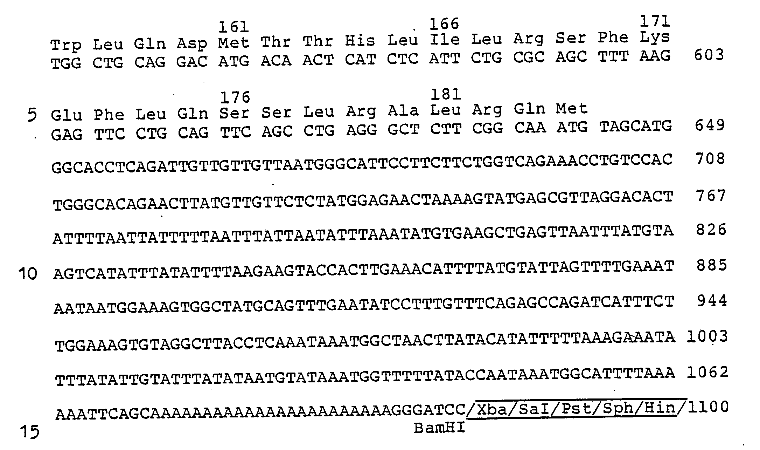

The EcoRI-BamHI 1,2 kbp BSF-2 cDNA insert was prepared by digestion of pBSF-2.38 (see Nature 324, 73-76 (1986)) with HindIII and BamHI. The obtained fragment containing a full length BSF-2 cDNA was then digested with HinfI and the recessed 3'-end filled in with Klenow fragment of DNA polymerase. After KpnI digestion, the obtained KpnI-HinfI 110 bp fragment containing the BSF-2 leader sequence was cloned into the multiple cloning site of pGEM4 digested previously with KpnI and SmaI. One of the selected clones was propagated and named as pBSF2-L8 (see Figure 3).

pBSF2-L8 was digested with BamHI and the recessed 3'-ends filled in with Klenow fragment of DNA polymerase. After the filling in of the BamHI site, the above mentioned HindIII-PstI FcεR cDNA was cloned into BamHI-PstI digested pBSF2-L8 as mentioned hereinbefore. One of the selected clones was propagated and named as psFcεR-1 (see Figure 4).

Surprisingly it was found, that when expressing such a coding sequence wherein at least a part of the cDNA sequence coding for the amino acids 1 to 148 is replaced by a suitable cDNA fragment coding for an eucaryotic signal sequence, in cells derived from multicellular organisms a highly bioactive water-soluble FcεR is obtained. Hereby, in principle, any cell culture is workable, whether using vertebrate or invertebrate cells. However, interest has been greatest in vertebrate cells, and propagation of vertebrate cells in culture (tissue culture) has become a routine procedure in recent years (Tissue Culture, Academic Press, Kruse and Patterson, Editors (1973)). Examples of such useful host cell lines are VERO and HeLa cells, Chinese hamster ovary (CHO) cell lines, and W138, BHK, COS-7 and MDCK cell lines. Expression vectors for such cells ordinarily include (if necessary) an origin of replication, a promotor located in front of the gene to be expressed, along with any necessary ribosome binding sites, RNA splice sites, polyadenylation site, and transcriptional terminator sequences.

For use in mammalian cells, the control functions on the expression vectors are often provided by viral genome. For example, commonly used promotors are derived from polyoma, Adenovirus 2, and most frequently Simian Virus 40 (SV40). The early and late promotors of SV40 are particularly useful because both are obtained easily from the virus as a fragment which also contains the SV40 viral origin of replication (Fiers et al., Nature 273, 113 (1978)). Smaller or larger SV40 fragments may also be used, provided there is included the approximately 250 bp sequence extending from the Hind III site toward the Bgl I site location in the viral origin of replication. Further, it is also possible, and often desirable, to utilize promotor or control sequences normally associated with the desired gene sequence, provided such control sequences are compatible with the host cell systems.

An origin of replication may be provided either by construction of the vector to include an exogenous origin, such as may be derived from SV40 or other viral (e.g., Polyoma, Adeno, VSV, BPV, etc.) source, or may be provided by the host cell chromosomal replication mechanism. If the vector is integrated into the host cell chromosome, the latter is often sufficient.

However, most preferably a cloning vehicle (shuttle plasmid) is used which enables replication in eukaryotes as well as in prokaryotes. The plasmid's ability to replicate in prokaryotes provides easy means for manipulating the DNA sequence and getting hold of large quantities of plasmid DNA needed for transfection into mammalian cells.

Such a shuttle plasmid contains prokaryotic DNA motifs as well as DNA sequences derived from eukaryotes.

The prokaryotic part of the plasmid consists of an origin of replication usually derived from the plasmid pBR322 (Mulligan R.C. et al. in Proc. Natl. Acad. Sci. USA 78, 2072-2076 (1981)) and a marker gene facilitating selection on antibiotic containing medium. The most widely used genes for selection are those mediating resistance to either ampicillin, tetracycline or chloramphenicol (Mulligan R.C. et al. in Proc. Natl. Acad. Sci. USA 78, 2072-2076 (1981)).

The eukaryotic part of the shuttle plasmid has to contain an origin of replication, usually derived from viral genomes such as Simian 40 Virus (Mulligan R.C. et al. in Proc. Natl. Acad. Sci. USA 78, 2072-2076 (1981)) or Bovine Papilloma Virus (DiMaio D. et al. in Mol. Cell. Biol. 4, 340-350 (1984)). Secondly a selectable marker gene is required to enable the cells harbouring the shuttle plasmid to grow under selective conditions in order to maintain the plasmid in the cells. This marker gene may be either of prokaryotic or of eukaryotic origin (e.g. prokaryotic genes: gpt gene coding for xanthine-guanine phosphoribosyltransferase (Mulligan R.C. et al. in Proc. Natl. Acad. Sci. USA 78, 2072-2076 (1981)), Mulligan R.C. et al. in Science 209, 1422 (1980)), neo gene coding for a bacterial phosphatase mediating resistance to the neomycin derivative G418 (Southern P. et al. in J. Mol. Appl. Genet. 1, 327 (1982), Scholer U. et al. in Cell 36, 1422 (1984), CAT gene coding for the chloramphenicol acetyl transferase (Gorman C. in Mol. Cell. Biol. 2, 1044 (1982)); eukaryotic genes: gene coding for the thymidine kinase (Wigler M. et al. in Cell 11, 223 (1977)). The third eukaryotic DNA motif enabling expression of the cloned gene of interest is a promoter sequence, which may be either constitutive or inducible (e.g. constitutive promoter: simian 40 virus or rous sarcoma virus (Mulligan R.C. et al. in Science 209, 1422 (1980), Laimons L. et al. in Proc. Natl. Acad. Sci. USA 79, 6453 (1982)); inducible promoter: mouse mammary tumor virus promoter (Chapman A.B. et al. in Mol. Cell. Biol. 3, 1421-1429, heat shock protein promoter (Pelham H. et al. in EMBO J. 1, 1473 (1982)-), metallothionein promoter (Mayo K. et al. in Cell 29, 99 (1982), Karin M. et al. in Nature 299, 797 (1982)).

One way to get hold of relatively high quantities of the soluble part of the $Fc_\epsilon$-receptor protein in higher eukaryotes is to anneal the soluble part of the $Fc_\epsilon$-receptor gene to the SV 40 promoter (constitutive) and clone this hybrid gene into a plasmid containing the gene coding for the dihydrofolate reductase (dhfr). Under selective pressure the dhfr gene and the adjacent DNA sequences are amplified up to a thousand times, elevating the yield not only of the dhfr gene but the soluble $Fc_\epsilon$-receptor part as well (EP-A-0 093 619).

A preferred embodiment of the present invention, however, is a vector suitable for expression in cells of multicellular organisms containing the above mentioned coding sequences of $psFc_\epsilon R$-1 as shown in Figure 4, the cells transfected with such a vector, the corresponding genes operably linked with a suitable replicon and control sequences, the expressed water-soluble $Fc_\epsilon R$ and processes of the preparation thereof.

For example, such a vector according to the invention can be prepared using as basic vector the PDE-2, pSV2 or $\pi$H3M vector as follows:

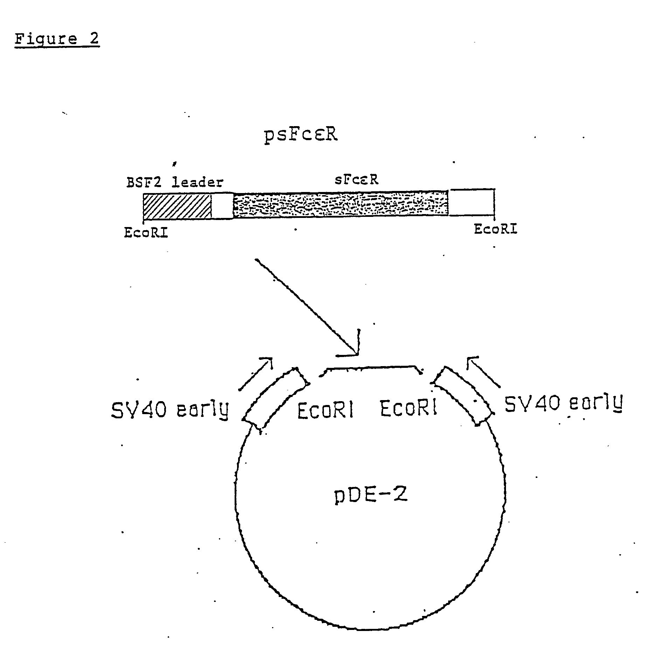

a) The expression vector PDE-2 (see Japanese Pat. Publication 1986/88879) which bears two SV40 early promotors was digested with EcoRI using standard techniques, and ligated with the EcoRI fragment of $psFc_\epsilon R$-1 containing the $BSF_2$ leader sequence (as shown on Figure 2). The resulting expression vector construct PDE-2s$Fc_\epsilon R$ was utilised for expression in monkey Cos-7 cells.

b) After digestion of the expression vector $\pi$H3M (see Proc. Natl. Acad. Sci. 84, 3365-3369 (1987)) with XhoI, the linearized plasmid was made blunt-ended with Klenow fragment in the presence of dNTP. Subsequently the vector was treated with bacterial alkaline phosphatase, extracted with phenol and precipitated.

In parallel the plasmid $psFc_\epsilon R$-1 (containing the soluble part of the $Fc_\epsilon$-receptor gene and the BSF-2 leader sequence upstream of the gene) was digested with EcoRI. After Klenow fill in reaction the DNA fragment was electroeluted.

The linearized, dephosphorylated $\pi$H3M vector was ligated to the BSF-2s$Fc_\epsilon$-DNA fragment. The resulting plasmid was transformed into E.coli and propagated and designated $\pi$s$Fc_\epsilon R$.

c) After digestion of the expression vector pSV2 gpt (see Science 209, 1422 (1980)) with EcoRI and BamHI, the obtained DNA was precipitated with ethanol and subsequently treated with the Klenow fragment. After inactivation of the enzyme, the DNA was treated with calf intestine alkaline phophatase, extracted with phenol, purified over agarose gel and electroeluted.

In parallel, the pBR322-dhfr plasmid containing the gene for the dihydrofolate reductase (dhfr) and the corresponding regulatory region derived from hamster cells (see Mol. Cell. Biol. 6, 425-440 (1986)) was digested with FspI and HindIII.

After ethanol precipitation the isolated 2658 bp DNA fragment was made blunt-ended and purified over agarose gel and electroeluated.

The thus obtained DNA fragment and the modified pSV2 gpt vector were ligated and transfected into E.coli JM101. After restriction enzyme analysis with EcoRI and MstII two positive clones differing in the orientations of the dhfr-gene in respect to SV40 ORI were selected and designated pSV2 gpt-dhfr17 resp. pSV2 gpt-dhfr20.

One of the obtained pSV2 gpt-dhfr vectors was digested with ApaI and treated with Klenow enzyme. After phenol/chloroform extraction and ethanol precipitation, the obtained DNA was incubated with HindIII and after inactivation of the enzyme with calf intestine alkaline phosphatase. The desired pSV2-dhfr vector, which does not contain the gpt gene, was obtained by extraction with phenol chloroform, by chromatography over agarose gel and by electroelution. The thus isolated pSV2-dhfr vector was ligated with a DNA fragment containing the coding sequence for the soluble part of $Fc_\epsilon R$ which upstream is ligated to the BSF-2 leader sequence. This fragment is preferably prepared by digestion of the $\pi$H3M plasmid containing the soluble part of the $Fc_\epsilon$-receptor gene which upstream is ligated to the BSF-2 leader sequence with PstI, incubation of the obtained reaction mixture with the Klenow enzyme in the presence of dNTP to create blunt ends, followed by phenol extraction and ethanol precipitation. The obtained DNA was incubated with HindIII, extracted with phenol chloroform and purified over agarose gel. After electroeluation of the isolated 1.500 bp fragment, the fragment was ligated with the linearized pSV2-dhfr vector and transfected into E.coli HB 110. After resctriction enzyme analysis two positive clones were selected and designated pSV2-dhfr17-s$Fc_\epsilon$ and pSV2-dhfr20-s$Fc_\epsilon$. The resulting expression vectors were utilized for expression in chinese hamster ovary cells.

In addition to microorganisms, cultures of cells derived from multicellular organisms may also be used as hosts. In principle, any such cell culture is workable, whether from vertebrate or invertebrate culture.

Propagation of cells in culture (tissue culture) has become a routine procedure (Tissue Culture, Academic Press, Kruse and Patterson, Editors (1973)). Examples of vertebrate host cell lines are VERO and Hela cells, Chinese hamster ovary (CHO) cell lines and WI38, BHK, Cos-7 and MDCK cell lines; but also invertebrate cell lines have become useful in recent years (e.g. 8f9 cloned cell line from Spodoptera frugiperda cells; available from ATCC).

Expression vectors for such cells ordinarily include (if necessary) an origin of replication, a promoter located in front of the gene to be expressed, along with any necessary ribosome binding sites, RNA splice sites, polyadenylation site, and transcriptional terminator sequences.

For use in mammalian cells, the control functions on the expression vectors are often provided by viral genome. For example, commonly used promoters are derived from polyoma, Adenovirus 2, and most frequently Simian Virus 40 (SV40); for invertebrates e.g. the promoter from the polyhedrin gene of Autographa californica nuclear polyhedrosis virus (AcNPV) (Summers M.D. and Smith G.E.; A Manual of Methods for Baculovirus Vectors and Insect Cell Culture Procedures (1987) Department of Entomology, Texas, Agricultural Experiment Station and Texas A & M University).

The early and late promoters of SV40 are particularly useful because both are obtained easily from the virus as a fragment which also contains the SV40 viral origin of replication (Fiers et al., Nature 273, 113 (1978)). Smaller or larger SV40 fragments may also be used, provided there is in cluded the approximately 250 bp sequence extending from the HindIII site toward the BglI site location in the viral origin of replication. Further, it is also possible, and often desirable, to utilise promoter or control sequences normally associated with the desired gene sequence, provided such control sequences are compatible with the host cell systems (e.g. the DMFR-gene for CHO-cells).

An origin of replication may be provided either by construction of the vector to include an exogenous origin, such as may be derived from SV40 or other viral (e.g., Polyoma, Adeno, VSV, BPV, AcNPV etc.) source, or may be provided by the host cell chromosomal replication mechanisms. If the vector is integrated into the host cell chromosome, the latter is often sufficient.

However, most preferably a cloning vehicle (shuttle plasmid) is used which enables replication in eukaryotes as well as in prokaryotes. The plasmids's ability to replicate in prokaryotes provides easy means for manipulating the DNA sequence and getting hold of large quantities of plasmid DNA needed for transfection into mammalian cells.

Such a shuttle plasmid contains prokaryotic DNA motifs as well as DNA sequences derived from eukaryotes.

The prokaryotic part of the plasmid consists of an origin of replication usually derived from the plasmid pBR322 (Mulligan R.C. et al. in Proc. Natl. Acad. Sci. USA 78, 2072-2076 (1981)) and a marker gene facilitating selection on antibiotic containing medium. The most widely used genes for selection are those mediating resistance to either ampicillin, tetracycline or chloramphenicol (Mulligan R.C. et al. in Proc. Natl. Acad. Sci. USA 78, 2072-2076 (1981)).

The eukaryotic part of the shuttle plasmid has to contain an origin of replication, usually derived from viral genomes such as Simian 40 Virus (Mulligan R.C. et al. in Proc. Natl. Acad. Sci. USA 78, 2072-2076 (1981)) or Bovine Papilloma Virus (DiMaio D. et al. in Mol. Cell. Biol. 4, 340-350 (1984)). Secondly a selectable marker gene is required to enable the cells harbouring the shuttle plasmid to grow under selective conditions in order to maintain the plasmid in the cells. This marker gene may be either of prokaryotic or or eukaryotic origin (e.g. prokaryotic genes: gpt gene coding for xanthine-guanine phosphoribosyltransferase (Mulligan R.C. et al. in Proc. Natl. Acad. Sci USA 78, 2072-2076 (1981), Mulligan R.C. et al. in Science 209, 1422 (1980)), neo gene coding for a bacterial phosphatase mediating resistance to the neomycin derivative G418 (Southern P. et al. in J. Mol. Appl. Genet. 1, 327 (1982), Scholer U. et al. in Cell 36, 1422 (1984)), CAT gene coding for the chloramphenicol acetyltransferase (Gormal C. in Mol. Cell. Biol. 2, 1044 (1982)), eukaryotic genes: gene coding for the thymidine kinase (Wigler M. et al. in Cell 11, 223 (1977)). The third eukaryotic DNA motif enabling expression of the cloned gene of interest is a promoter sequence, which may be either constitutive or inducible (e.g. constitutive promoter; simian 40 virus or rous sarcoma virus (Mulligan R.C. et al. in Science 209, 1422 (1980), Laimons L. et al. in Proc. Natl. Acad. Sci. USA 79, 6453 (1982)); inducible promoter: mouse mammary tumor virus promoter (Chapman A.B. et al. in Mol. Cell. Biol. 3, 1421-1429), heat shock protein promoter (Pelham H. et al. in EMBO J. 1, 1473 (1982)), metallothionein promoter (Mayo K. et al. in Cell 29, 99 (1982), Karin M. et al. in Nature 299, 797 (1982)).

Transfer of foreign DNA into appropriate host cells can be managed by microinjection of DNA directly into the cell nucleus of the host cell (Capecchi M. in Cell 22, 479 (1980)); protoplast fusion of the bacterial protoplast carrying foreign DNA with the eucaryotic host cell (Schaffer W. in Proc. Natl. Acad. Sci. USA, 77, 2163 (1980)); electroporation of the host cell membrane in the presence of DNA being transfected (Neuman E. et al. in EMBO J., 1, 841 (1982); fusion of liposomes containing recombinant DNA with host cells (Fraley R. et al. in J. Biol. Chem. 255, 10431 (1980)). Most frequently used methods are the co-precipitation between DNA and calcium phosphate (Graham, F.L. and van der Erb., A.J. in Virology 52, 456 (1973)) and DEAE-dextran mediated transfection (Vaheri A. et al. Virology 27, 435 (1965)).

After incubation the transfected cos-7 or CHO cells, the culture supernatants were collected to test for soluble Fc$_\epsilon$R as follows:

The Cos cell supernatants were tested on an eosinophilic cell line, EoL-3 which expresses Fc$_\epsilon$R, the presence of such receptors have been tested utilising monoclonal antibodies specific to Fc$_\epsilon$R (8-30) as described in European Patent Application No. 87110658.9 of the same applicant. As shown on Figure 5, these cells stain for Fc$_\epsilon$R with 8-30 antibodies compared to the controls.

Furthermore, Fc$_\epsilon$R on cells are detected by an assay with the use of fixed ox RBC (ORBS) coated with human IgE (Gonzalez-Molina, A. and Spiegelberg, H.L. J. Clin. Invest. 59, 616 (1977)). The number of IgE rosette-forming cells is estimated after subtracting the number of non-specific binding with fixed 0RBC coated with bovine serum albumin.

The IgE-binding activity found in the PDE-2sFc$_\epsilon$R transfected Cos cell supernatant was analysed by its inhibitory activity on the binding (rosette-formation) of IgE-0RBC to the EoL-3 cells. The results shown on Table I indicate that the soluble Fc$_\epsilon$R is capable of inhibiting competitively the binding of IgE to its receptors.

The presence of soluble receptor activity was confirmed by the ELISA assay as described below:

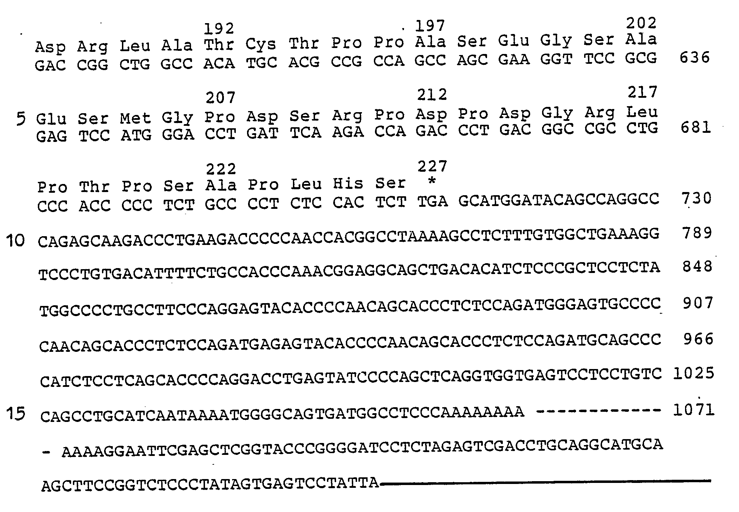

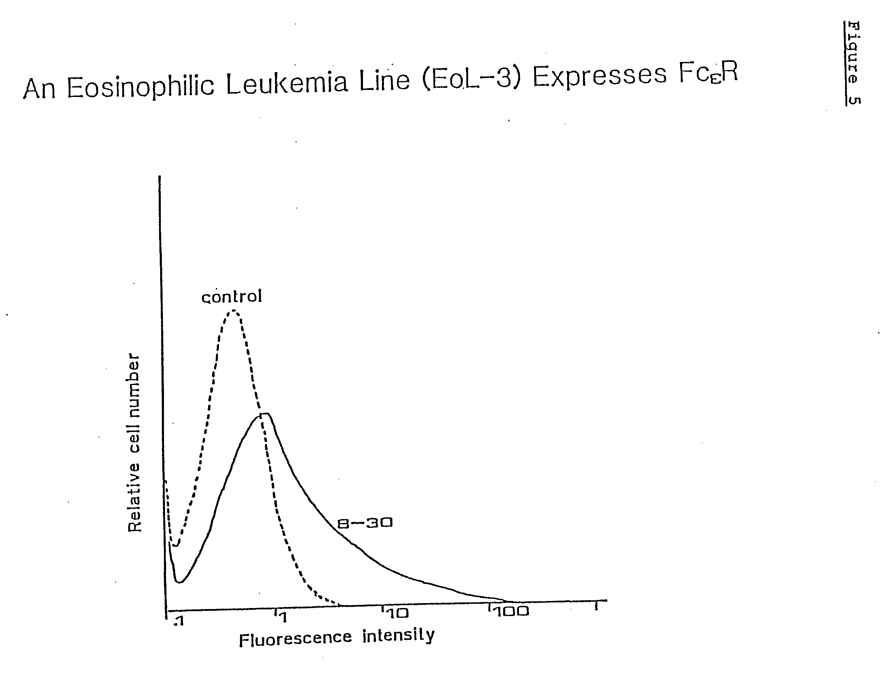

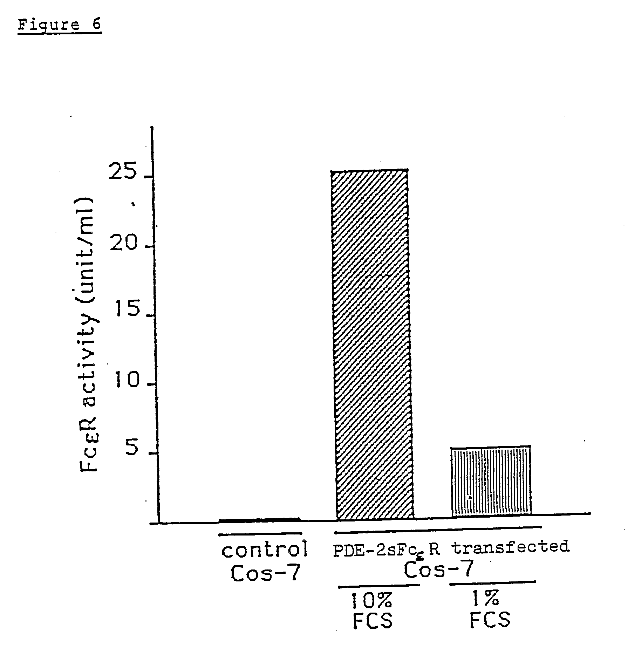

The Fc$_\epsilon$R activity was measured by its ability to bind to the monoclonal antibodies 3-5 and 8-30 and monitored utilising a double antibody enzyme-linked immunosorbent assay (ELISA). Whereas no Fc$_\epsilon$R activity was detected in the supernatants of control Cos-7 cells, there was significant secretion of receptor activity in the PDE-2sFc$_\epsilon$R-transfected Cos-7 cells. In fact, the level of Fc$_\epsilon$R activity was 5 and 25 units/ml in transfectant culture supernatants containing 1 % and 10 % fetal calf serum (FCS) respectively. The results of Fc$_\epsilon$R activity are shown on Figure 6.

Similar results were obtained when using water-soluble Fc$_\epsilon$R isolated from transfected CHO cells.

Moreover, it is known, that immediate-type hypersensitivity can be manifested as a broad spectrum of symptoms ranging from seasonal rhinitis to anaphylaxis. The mechanism of immediate hypersensitivity involves IgE antibodies and reactive cells such as tissue mast cells and basophils which bear on their surface specific receptors for these antibodies. Upon triggering of those cells by specific allergens, a cascade of biochemical events lead to the release of preformed mediators from the cellular granules and newly synthesized lipid metabolites of membrane arachidonic acid, these agents cause an inflammatory process characterised by increased vascular permeability, smooth muscle contraction and all the symptoms associated with such a reaction. The process is amplified by the chemotaxis of cells such as neutrophils and eosinophils which in turn will enhance the reaction by releasing their own proinflammatory components. A family of factors specific for the eosinophils are the eosinophil chemotactic factors of anaphylaxis, these are acidic tetrapeptides and attract eosinophils to the site of the allergic reaction. More recent findings indicate the importance of eosinophils in allergy and in parasitic infestations. It has been demonstrated that the eosinophils bear on their surface receptors for IgE ($Fc_\epsilon R$) which in the presence of IgE can actively participate in IgE-mediated events characterised by degranulation and further amplification of the inflammatory process. Furthermore, the $Fc_\epsilon R$ found on eosinophils is of low affinity compared to that found on mast cells and basophils. In view of the mounting importance of eosinophils in immediate-type hypersensitivity and particularly asthma it is important to find means to control the IgE-mediated degranulation of these cells. Moreover, the central issue is to prevent or compete with the binding of IgE to mast cells, basophils and eosinophils. It has been hypothesised that IgE-binding factors might be able to achieve that either by controlling IgE biosynthesis or by neutralising or scavenging serum IgE.

The before mentioned evidence shows that the water-soluble $Fc_\epsilon R$ prepared according to the present invention binds IgE and in its soluble form it can prevent the binding of IgE to eosinophils which are known to be involved in certain aspects of immediate hypersensitivity (allergy) and particularly asthma.

Therefore, the soluble $Fc_\epsilon R$ prepared according to the invention is useful in controlling the inflammatory effects of allergy and is suitable for the treatment or prophylaxis of local and allergic reactions induced by IgE, which is a further object of the invention.

The soluble $Fc_\epsilon R$ may be incorporated for the pharmaceutical use in the suitable pharmaceutical compositions, such as solutions or sprays.

The following examples, which are not exhaustive, will illustrate the invention in greater detail:

### Example A

The monoclonal anti-$Fc_\epsilon R$ antibodies 3-5 ($\gamma_1$) and 8-30 ($\mu$) were produced by hybridization of P3U1 myeloma with spleen cells from Balb/c mice immunized with RPMI-8866 cells (see European Patent Application No. 86 110 420.6 of the same Applicant, filed on July 29, 1986). The 8-30 antibody recognizes the epitope close to IgE binding site of $Fc_\epsilon R$ and can block binding of IgE to 8866 lymphoblastoid cells. The 3-5 antibody, recognizes a different epitope on $Fc_\epsilon R$ and can not block effectively IgE binding to its receptors. These antibodies precipitate 46 kd and 25 kd polypeptides under reducing and non reducing conditions. The monoclonal antibodies were purified from ascitis by 50 % saturated ammonium sulfate precipitation followed by gel filtration using Sepharose 6B (Pharmacia Fine Chemical, Uppsala, Sweden) for IgM class or ion exchange chromatography using QAE-Sephadex (Pharmacia Fine Chemical) for IgG1. The polyclonal mouse IgG was isolated in the same fashion. The anti-mouse IgM-alkaline phosphatase conjugate was purchased from Tago (Burlingame, CA).

### Example B

#### Construction of plasmid psFc$_\epsilon$R-1

a) 350 $\mu$g of pBSF2-38 (see Nature 324, 73-76 (1986)), which contains the BSF-2 cDNA in the SmaI site of pGEM4, were digested with 700 units of EcoRI and BamHI in 500 $\mu$l of a high salt buffer (100 mM NaCl, 50 mM Tris-HCl, pH 7.5, 10 mM MgCl$_2$, 1 mM DTT) for 2 hrs at 37°C. The digested DNA was applied on a preparative 1 % agarose gel electrophoresis and the EcoRI-BamHI fragment containing the full-length 1.2 kbp BSF-2 cDNA was electroeluted from the gel, precipitated with 70 % ethanol and dissolved at a concentration of 1 $\mu$g/$\mu$l in TE buffer. 20 $\mu$g of this fragment were digested with 40 units of HinfI in 50 $\mu$l of a high salt buffer for 1 hr, phenol-extracted and ethanol-precipitated. The digested DNAs were dissolved in 25 $\mu$l of 1 x nick translation buffer (50 mM Tris-HCl, pH 7.2, 10 mM MgSO$_4$, 0.1 mM DTT, 50 $\mu$g/ml BSA) and incubated with 1 ml of 8.0 units/$\mu$l Klenow fragment and 1 mM dNTP at 20°C for 30 minutes. The filling in reaction was terminated by incubation at 70°C for 5 min. The resulting 127 bp blunt ended fragment was phenol-extracted, digested with 40 units KpnI in 50 $\mu$l of a low salt buffer (10 mM Tris-HCl, pH 7.5, 10 mM MgCl$_2$, 1 mM DTT) for 1 hr at 37°C, incubated with 2.5 units bacterial alkaline phosphatase at 65°C for 30 min and then applied on 1 % preparative agarose gel and electrophoresed. The 110 bp fragment containing the BSF-2 leader sequence was electroeluted and ethanol-precipitated. - The 110 bp fragment was dissolved in 10 $\mu$l of ligation buffer (50 mM Tris-HCl, pH 7.4, 10 mM MgCl$_2$, 10 mM DTT, 1 mM spermidine, 1 mM ATP, 0.1 mg/ml BSA) and ligated with 1 $\mu$g of KpnI and SmaI digested pGEM4 by incubating with 200 units of T4 ligase at 4°C for 16 hrs and transfected into E.coli (MC1065). From the obtained colonies four colonies were picked up, one clone was selected, propagated and after confirmation that the plasmid of this selected clone contained only one leader sequence, it was named as pBSF2-L8 (see Figure 3).

b) 80 $\mu$g of plasmid LE-392 or pGEM4(pFc$_\epsilon$R-1) were digested with 150 units of HindIII in 200 $\mu$l of a low salt buffer (10 mM Tris-HCl, pH 7.5, 10 mM MgCl$_2$, 1 mM DTT) at 37°C for 1 hr and applied on 1 % preparative agarose gel electrophoresis. The HindIII fragment containing the soluble Fc$_\epsilon$R region was electroeluted from the gel, ethanol-precipitated and dissolved at a concentration of 1 $\mu$g/$\mu$l in TE buffer. 1 $\mu$g of the HindIII fragment was incubated with 8.2 units Klenow fragment and 1 mM dNTP in 10 $\mu$l of 1 x nick translation buffer at 20°C for 30 min to fill in the recessive 3'-ends, phenol-extracted and ethanol-precipitated. The HindIII fragments, the 3'-ends of which had been filled in, were digested with 2 units PstI in 10 $\mu$l of the medium salt buffer (50 mM NaCl, 10 mM Tris-HCl, pH 7.5, 10 mM MgCl$_2$, 1 mM DTT) at 37°C for 1 hr, incubated with 0.25 unit bacterial alkaline phosphatase at 65°C for 30 min and ethanol-precipitated. Separately, 1 $\mu$g of pBSF2-L8 was digested with 2 units BamHI in 20 $\mu$l of a high salt buffer at 37°C for 1 hr, phenol-extracted and ethanol-precipitated. The BamHI-digested pBSF2-L8 was dissolved in 10 $\mu$l of 1 x nick translation buffer and incubated with 8.0 units Klenow fragment and 1 mM dNTP at 20°C for 30 min to fill in the recessive 3'-ends, phenol-extracted and ethanol-precipitated. The precipitate was dissolved in 20 $\mu$l of a high salt buffer, digested with 2 units PstI at 37°C for 1 hr, phenol-extracted and ethanol-precipitated. - The PstI-digested fragment containing the soluble Fc$_\epsilon$R coding region and PstI digested pBSF2-L8 were ligated by incubating with 200 units T4 ligase in 10 $\mu$l of ligation buffer at 4°C for 16 hrs and transfected into E.coli (MC1065). Eight colonies were picked up from the obtained colonies. One clone was selected, propagated and after confirmation of the plasmid construction named psFc$_\epsilon$R-1. The propagated psFc$_\epsilon$R-1 contains seven bases from the multiple cloning into pGEM4 between BSF-2 leader and Fc$_\epsilon$R sequences in frame (see Figure 1: nucleotides 137 to 143).

## Example C

### Construction of pSV2-gpt-dhfr plasmids

a) 5 $\mu$g of the expression vector pSV2 gpt (see Science 209, 1422 (1980)) were digested with EcoRI and BamHI in a solution containing 100 mM NaCl, 10 mM Tris-HCl (pH 7,5), 6 mM MgCl$_2$, 100 $\mu$g/ml gelatine and 6 mM $\beta$-mercaptoethanol at 37°C over night. After precipitation of the DNA with ethanol at -70°C the DNA was incubated with 5 units of Klenow fragment in a solution containing of 7 mM MgCl$_2$, 7 mM Tris-HCl (pH 7,5), 50 mM NaCl, 1 mM DTT and 50 $\mu$M dNTP for 15 minutes at 37°C. After inactivation of the enzyme at 68°C for 10 minutes 1/10 volume of Tris-HCl (pH 8,0) and 20 units of calf intestine alkaline phosphatase were added. Incubation was performed at 37°C for 2 hours. Subsequently the solution containing the desired DNA was extracted twice with phenol and applied on a 1 % agarose gel. The modified linearized plasmid was electroeluted.

b) 5 $\mu$g of pBR322-dhfr plasmid (see Mol. Cell. Biol. 6, 425-440 (1986)), containing the gene for the dhfr (dihydrofolate reductase) and the corresponding regulatory region derived from hamster cells, were digested with 25 units of FspI and 60 units of HindIII in 100 $\mu$l of a solution containing 10 mM Tris-HCl (pH 7,5), 50 mM NaCl, 6 mM MgCl , 6 mM $\beta$-mercaptoethanol and 100 $\mu$g/ml gelatine over night at 37°C. After ethanol precipitation the generated DNA fragment (2658 base pairs) was made blunt-ended and purified as described above.

c) The dhfr fragment and the modified pSV2 gpt vector were ligated in 10 $\mu$l of ligase buffer (40 units ligase, 50 mM Tris-HCl (pH 7,5), 10 mM MgCl$_2$, 20 mM DTT, 1 mM ATP, 50 $\mu$g/ml BBA) at 14°C over night and used for transformation into E.coli JM 101. After restriction enzyme analysis with EcoRI and MstII two positive clones differing in the orientation of the dhfr-gene (in respect to SV40 ORI) were selected. These vectors were designated pSV2-gpt-dhfr17 resp. pSV2-gpt-dhfr20.

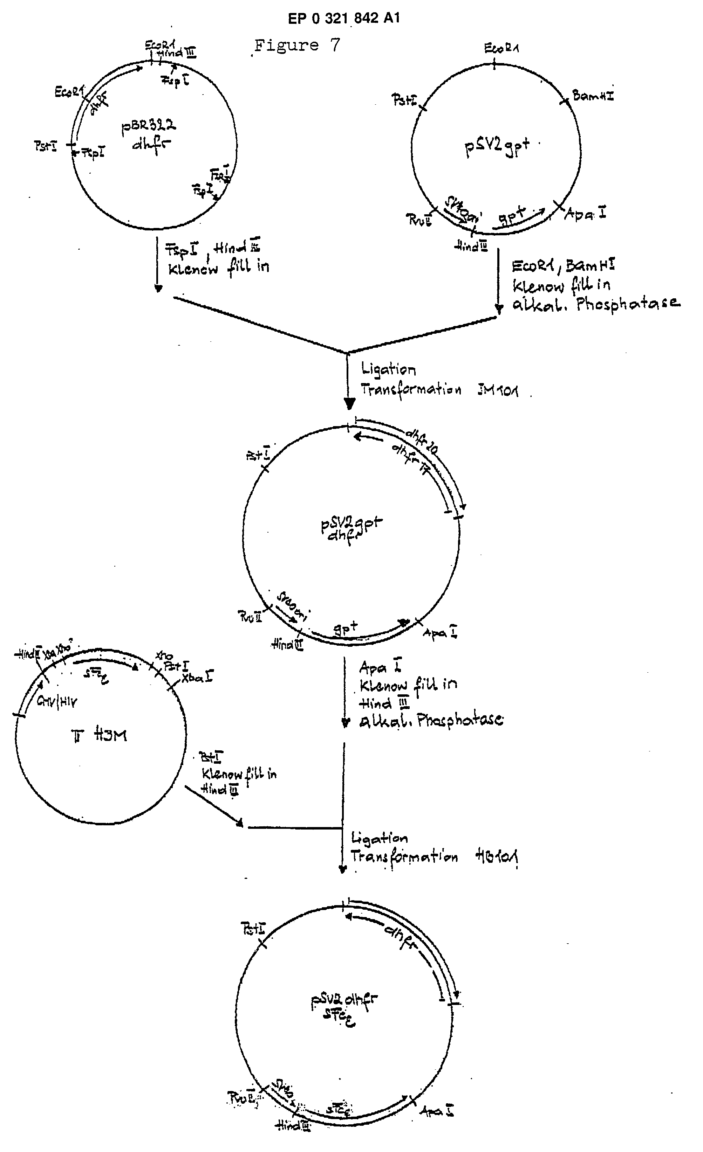

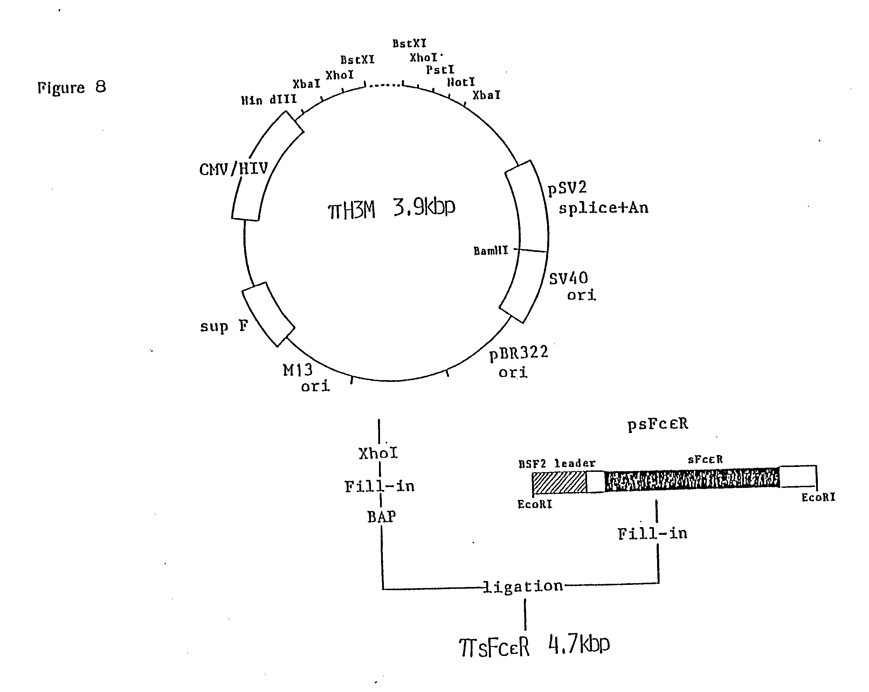

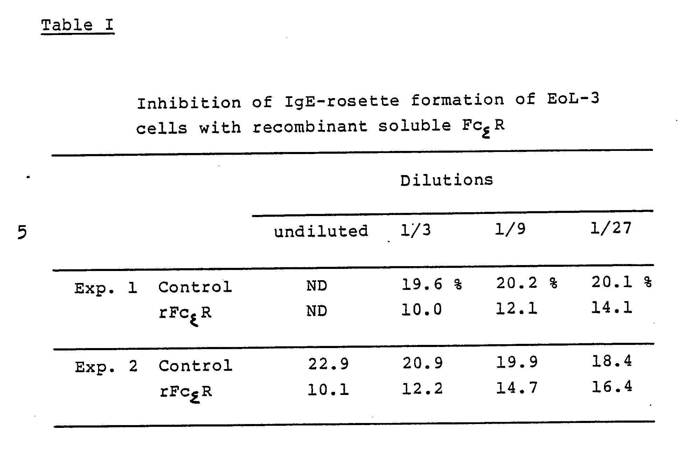

d) 5 $\mu$g of one of the pSV2-gpt-dhfr plasmids were digested with 20 units of ApaI in 100 mM NaCl, 10 mM Tris-HCl (pH 7,5), 6 mM MgCl$_2$, 6 mM ß-mercaptoethanol and 100 $\mu$g/ml gelatine for 2 hours at 37°C. After Klenow fill in reaction for 25 minutes at 20°C with 5 units Klenow enzyme in the same reaction mixture and the presence of 5 $\mu$M of dNTP the DNA was extracted with phenol/chloroform and finally collected by ethanol precipitation. The obtained DNA was dissolved in 10 $\mu$l of 100 mM NaCl, 10 mM Tris-HCl (pH 7,5), 6 mM MgCl$_2$, 6 mM $\beta$-mercaptoethanol and 100 $\mu$g/ml gelatine and incubated with 20 units of HindIII at 37°C for 2 hours. After inactivation of the enzyme at 70°C for 10 minutes 20 units of calf intestine alkaline phosphatase were added and the reaction mixture was further incubated for 30 minutes at 37°C. The mixture containing the desired DNA was extracted with phenol/chloroform two times and finally applied to a 1 % agarose gel. The pSV2-dhfr vector, which does not contain the gpt gene, was purified by electroelution (see Figure 7).

Example 1

Construction of PDE-2sFc$_\epsilon$R

20 $\mu$g of the expression vector PDE-2 (see Japanese Patent Publication 1986/88879) were digested with 40 units of EcoRI in a total volume of 100 $\mu$l containing 50 mM NaCl, 100 mM Tris-HCl pH 7.5, 5 mM MgCl$_2$ and 100 $\mu$g/ml BSA at 37°C for two hours. Following extraction with phenol chloroform the DNA was recovered by ethanol precipitation. The digested DNA was dissolved in 25 $\mu$l and was treated with 20 units of bacterial alkaline phosphatase at 65°C for 30 minutes and phenol/chloroform extracted twice and ethanol precipitated. In parallel an EcoRI fragment of psFc$_\epsilon$R-1 plasmid (see Figure 4) was produced as follows: 20 $\mu$g of psFc$_\epsilon$R-1 were digested with 20 units of EcoRI in 100 $\mu$l of 50 mM NaCl, 100 mM Tris-HCl pH 7.5, 5 mM MgCl$_2$ and 100 $\mu$g/ml BSA at 37°C for two hours. Both the vector and the insert DNA were applied on 1 % preparative agarosegel and electrophoresed. The 1,5 kb EcoRI fragment of psFc$_\epsilon$R-1 (consisting of the BSF-2 leader and sFc$_\epsilon$-sequence) and the linearised PDE-2 vector were electroeluted and ethanol precipitated. The dephosphorylated linearised vector was ligated with the 1,5 kb EcoRI fragment by incubating with 200 units of T4 ligase in 20 $\mu$l of ligation buffer (50 mM Tris-HCl pH 7.4, 10 mM MgCl$_2$, 20 mM DTT, 1 mM spermidin, 1 mM ATP and 100 $\mu$g.ml BSA) at 4°C for 16 hours and transfected into E.coli. Restriction enzyme analysis was performed to identify the correct insert. One positive clone was selected and named as pDE-2sFc$_\epsilon$R. The resulting expression vector PDE-2sFc$_\epsilon$R was utilized for expression in monkey Cos-7 cells according to known methods.

Example 2

Construction of $\pi$sFc$_\epsilon$R

After digestion of 5 $\mu$g of the expression vector $\pi$H3M (see Proc. Natl. Acad. Sci. 84, 3365-3369 (1987)) with 20 units of XhoI for two hours at 37°C in a solution containing 50 mM NaCl, 10 mM Tris-HCl (pH 7,5), 6 mM MgCl$_2$, 6 mM $\beta$-mercaptoethanol and 100 $\mu$g/ml gelatine, the linearized plasmid was made blunt-ended with Klenow fragment in the presence of 5 $\mu$M of dNTP at 20°C for 25 minutes. Subsequently the vector was treated with bacterial alkaline phosphatase, extracted with phenol and precipitated.

In parallel 5 $\mu$g of the plasmid psFc$_\epsilon$R-1 (containing the soluble part of the Fc$_\epsilon$-receptor gene and the BSF-2 leader sequence upstream of the gene) were digested with 20 units of EcoRI at 37°C for 2 hours in the buffer as described above. After Klenow fill in reaction the DNA fragment was electroeluted from an agarose gel. The linearized, dephosphorylated $\pi$H3M vector was ligated to the BSF-2/sFc$_\epsilon$-DNA fragment in 20 $\mu$l of 50 mM Tris-HCl (pH 7,5), 10 mM MgCl$_2$, 20 mM DTT, 1 mM ATP, 50 $\mu$g/ml BSA and 40 units of T4 DBA ligase at 14°C over night. The resulting plasmid was transformed into E.coli and propagated.

Example 3

Expression of PDE-2sFc$_\epsilon$R

Cos-7 cells (5 x 10$^5$ cells per plate, diameter 60 mm) were seeded onto 60 mm plates one day prior to transfection. Transfection was carried out with 2 $\mu$l of plasmid DNA in 1 ml of 25 mM Tris-HCl (pH 7.5), 137 mM NaCl, 5 mM KCl, 0,6 mM Na$_2$HPO$_4$, 0,5 mM MgCl$_2$, 0,7 mM CaCl$_2$ and 500 $\mu$g of DEAE-dextran (Pharmacia Fine Chemical). After 1 hour incubation at 37°C, the solution was replaced with DMEM containing 10 % FCS and 150 $\mu$M chloroquine, incubated at 37°C for 3 hours and then replaced with fresh DMEM containing 10 % FCS. One day after transfection the medium was changed to contain 1 % or 10 % FCS in DMEM. The culture supernatants were collected 2 days following the medium change and tested for soluble Fc$_\epsilon$R activity.

Similar results were obtained with $\pi$sFc$_\epsilon$R transfected Cos-7 cells.

Example 4

IgE rosette formation and its inhibition

For IgE rosette inhibition, 25 $\mu$l of Fc$_\epsilon$R bearing cells (5x10$^6$/ml) are mixed with a specific volume (e.g. 100 $\mu$l) of test sample or control medium and incubated for 1 hour at 4°C. The number of rosettes with 3 or more ORBC are counted. The results are shown in Table I. In experiments I and II the Fc$_\epsilon$R bearing cells are the EoL-3 cells. The control medium is the supernatant of the control Cos-7 cells, i.e. non-transformed cells.

Example 5

## Enzyme Linked Immunosorbent Assay (ELISA)

96 well mictrotiter plates were initially coated with the monoclonal antibody 3-5 100 $\mu$l/well (10 $\mu$g/ml) in coating buffer (NaHCO$_3$ 0.1 M, pH 9.6), and incubated overnight at 4°C. The plates were then washed 4 times with rinse buffer, i.e. Dulbecco's phosphate buffer pH containing 0,05 % Tween 20, followed by the addition of 100 $\mu$l test sample diluted with diluent buffer (Tris-HCl 0,05 M, pH 8.1, MgCl$_2$ 1 mM, NaCl 0,15 M, Tween 20 0,05 % (v/v), NaN$_3$ 0,02 %, BSA 1 %). The microtiter plates were incubated for 2 hours at room temperature, and washed 4 times with rinse buffer, followed by the addition of 100 $\mu$l of pretitrated and diluted goat-anti-mouse IgM-alkaline phosphatase conjugates. The plates were incubated for two hours at room temperature and washed 4 times with rinse buffer. In the final step 100 $\mu$l of substrate, p-phenyl phosphate disodium (1 mg/ml) in substrate buffer (NaHCO$_3$ 0,05M, pH 9.8, MgCl$_2$ x 6 H$_2$O, 10 mM) was added and the colorimteric reaction measured every 30 minutes for two hours at 405 and 620 nm. The results of Fc$_\epsilon$R activity are shown on Figure 6.

## Example 6

## Construction of pSV2-dhfr-sFc$_\epsilon$

10 $\mu$g of the $\pi$H3M plasmid (containing the soluble part of the Fc$_\epsilon$-receptor gene which upstream is ligated to the BSF-2 leader sequence) were digested with PstI in 50 mM NaCl, 10 mM Tris-HCl (pH 7,5), 6 mM MgCl$_2$, 6 mM $\beta$-mercaptoethanol and 100 $\mu$g/ml gelatine for 2 hours at 37°C. After incubating the reaction mixture with 5 units of Klenow enzyme for 25 minutes at 20°C in the presence of 50 $\mu$M of dNTP to create blunt ends, the DNA was extracted with phenol and precipitated with ethanol. The obtained DNA was dissolved in 20 $\mu$l of a solution consisting of 100 mM NaCl, 10 mM Tris-HCl (pH 7,5), 6 mM MgCl$_2$, 6 mM $\beta$-mercaptoethanol and 100 $\mu$g/ml gelatine and incubated with 20 units of HindIII at 37°C for 2 hours. After phenol/chloroform extraction the reaction mixture containing the desired DNA was applied on a 1 % agarose gel and a 1500 base pair fragment was electroeluted. After ligation of the 1500 bp fragment into the linearized pSV2-dhfr vector (lacking the gpt gene) in 10 $\mu$l of 50 mM Tris-HCl (pH 7,5), 10 mM MgCl$_2$, 20 mM DTT, 1 mM ATP and 50 $\mu$g/ml BSA and 40 units of T4 DNA ligase at 14°C over night the ligation mixture was used to transform E.coli HB 101. After restriction enzyme analysis a positive clone was selected and designated pSV2-dhfr-sFc$_\epsilon$ (see Figure 7).

## Example 7

## Expression of pSV2-dhfr-sFc$_\epsilon$

Chinese hamster ovary cells deficient in dihydrofolate reductase (dhfr$^-$, Chasin, L.) were propagated in $\alpha$-MEM medium containing 10 % foetal calf serum, Hypoxanthine and Thymidine (HT). Transfection of the plasmid pSV2-dhfr17-sFc$_\epsilon$ or pSV2-dhfr20-sFc$_\epsilon$ into CHO cells was performed by the calcium phosphate precipitation method (see Virology 52, 456 (1973)). 7 x 10$^5$ cells were seeded on culture plates one day prior transfection. Cells were exposed to a calcium phophate precipitate containing 10 $\mu$g of plasmid DNA for 4 hours at 37°C. Subsequently the medium was aspirated and replaced by selection medium $\alpha$-MEM with 10 % foetal calf serum, which was changed every 2 days. Colonies of transformed cells appeared 12 to 16 days later and were isolated with cloning cylinders or pipette tips. Supernatants were tested soluble for Fc$_\epsilon$R activity by ELISA assays.

## Claims

1. A cloned gene operably linked to a replicon and control sequence both suitable for expression in cells of multicellular organisms wherein at least a part of the coding sequence for the amino acids 1 to 148 of the whole Fc$_\epsilon$R-gene as shown in Figure 1 is replaced by an eucaryotic signal sequence or a coding equivalent thereof.

2. The cloned gene of claim 1 wherein said signal sequence is an interleukin cDNA signal sequence, preferably the BSF-2 signal sequence, which is comprised, for example, by the 110 bp KpnI-HinfI fragment of the plasmid pBSF-2.38, or a coding equivalent thereof.

3. An expression vector suitable for expression in cells of multicellular organisms containing a cloned gene as claim in claim 1 or 2 or a coding equivalent thereof.

4. A cell of a multicellular organism transfected with an expression vector as claimed in claim 3.

5. Water-soluble part of human low affinity Fc$_\epsilon$-receptor coded by a DNA-molecule as claimed in claim 1 or 2 and the O-glycosylated derivate thereof.

6. Water-soluble part of human low affinity Fc$_\epsilon$-receptor as claimed in claim 5 wherein said Fc$_\epsilon$-receptor is expressed by a cell line as claimed in claim 4 and the O-glycosylated derivate thereof.

7. Water-soluble part of human low affinity $Fc_\epsilon$-receptor as claimed in claim 6 wherein the expressed peptide has the formula as shown in Figure 4 and the O-glycosylated derivate thereof.

8. Pharmaceutical composition containing a water-soluble $Fc_\epsilon$-receptor as claimed in any of the claims 5 to 7.

9. Use of the water-soluble $Fc_\epsilon$-receptor as claimed in any of the claims 5 to 7 for the preparation of a pharmaceutical composition.

10. A method of preparing a cloned gene as claimed in claim 1 or 2 which comprises ligating a DNA-sequence coding at least the water-soluble part of human low affinity receptor starting with the amino acid 150 as shown in Figure 1 with an eucaryotic signal sequence.

11. A method of preparing an expression vector as claimed in claim 3 which comprises ligating a suitable linearised vector with a cloned gene as claim in claim 3.

12. A method of preparing a cell line as claimed in claim 4 which comprises transfecting a cell with an expression vector as claimed in claim 3.

13. A method of preparing water-soluble human low affinity $Fc_\epsilon$-receptor as claimed in any of the claims 5 to 7 comprising culturing a transfected cell as claimed in claim 4 and isolating the water-soluble $Fc_\epsilon$-receptor as produced.

14. Preparation of a pharmaceutical composition as claimed in claim 8 which comprises incorporating an effective amount of a water-soluble $Fc_\epsilon$-receptor as claimed in any of the claims 5 to 7 in one or more excipients.

Figure 1: Scheme of pFc_εR-1

```
                                                          EcoRI
_____ GAATTCCCTCCTGCT          8
```

TAAACCTCTGTCTCTGACGGTCCCTGCCAATCGCTCTGGTCGACCCCAACACACTAGGA          67

GGACAGACACAGGCTCCAAACTCCACTAAGTGACCAGAGCTGTGATTGTGCCCGCTGAG          126

TGGACTGCGTTGTCAGGGAGTGAGTGCTCCATCATCGGGAGAATCCAAGCAGGACCGCC          185

```
                      5                     10                     15
Met Glu Glu Gly Gln Tyr Ser Glu Ile Glu Glu Leu Pro Arg Arg
ATG GAG GAA GGT CAA TAT TCA GAG ATC GAG GAG CTT CCC AGG AGG          230

                         20                     25                     30
Arg Cys Cys Arg Arg Gly Thr Gln Ile Val Leu Leu Gly Leu Val
CGG TGT TGC AGG CGT GGG ACT CAG ATC GTG CTG CTG GGG CTG GTG          275

                         35                     40                     45
Thr Ala Ala Leu Trp Ala Gly Leu Leu Thr Leu Leu Leu Leu Trp
ACC GCC GCT CTG TGG GCT GGG CTG CTG ACT CTG CTT CTC CTG TGG          320

                         50                     55                     60
His Trp Asp Thr Thr Gln Ser Leu Lys Gln Leu Glu Glu Arg Ala
CAC TGG GAC ACC ACA CAG AGT CTA AAA CAG CTG GAA GAG AGG GCT          365

                         65                     70                     75
Ala Arg Asn Val Ser Gln Val Ser Lys Asn Leu Glu Ser His His
GCC CGG AAC GTC TCT CAA GTT TCC AAG AAC TTG GAA AGC CAC CAC          410

                         80                     85                     90
Gly Asp Gln Met Ala Gln Lys Ser Gln Ser Thr Gln Ile Ser Gln
GGT GAC CAG ATG GCG CAG AAA TCC CAG TCC ACG CAG ATT TCA CAG          455

                         95                     100                     105
Glu Leu Glu Glu Leu Arg Ala Glu Gln Gln Arg Leu Lys Ser Gln
GAA CTG GAG GAA CTT CGA GCT GAA CAG CAG AGA TTG AAA TCT CAG          500

                         110                     115                     120
Asp Leu Glu Leu Ser Trp Asn Leu Asn Gly Leu Gln Ala Asp Leu
GAC TTG GAG CTG TCC TGG AAC CTG AAC GGG CTT CAA GCA GAT CTG          545

                         125                     130                     135
Ser Ser Phe Lys Ser Gln Glu Leu Asn Glu Arg Asn Glu Ala Ser
AGC AGC TTC AAG TCC CAG GAA TTG AAC GAG AGG AAC GAA GCT TCA          590

                         140                     145                     150
Asp Leu Leu Glu Arg Leu Arg Glu Glu Val Thr Lys Leu Arg Met
GAT TTG CTG GAA AGA CTC CGG GAG GAG GTG ACA AAG CTA AGG ATG          635

                         155                     160                     165
Glu Leu Gln Val Ser Ser Gly Phe Val Cys Asn Thr Cys Pro Glu
GAG TTG CAG GTG TCC AGC GGC TTT GTG TGC AAC ACG TGC CCT GAA          680
```

```
                        170                        175                        180
     Lys Trp Ile Asn Phe Gln Arg Lys Cys Tyr Tyr Phe Gly Lys Gly
     AAG TGG ATC AAT TTC CAA CGG AAG TGC TAC TAC TTC GGC AAG GGC   725

                        185                        190                        195
     Thr Lys Gln Trp Val His Ala Arg Tyr Ala Cys Asp Asp Met Glu
     ACC AAG CAG TGG GTC CAC GCC CGG TAT GCC TGT GAC GAC ATG GAA   770

                        200                        205                        210
     Gly Gln Leu Val Ser Ile His Ser Pro Glu Glu Gln Asp Phe Leu
     GGG CAG CTG GTC AGC ATC CAC AGC CCG GAG GAG CAG GAC TTC CTG   815

                        215                        220                        225
     Thr Lys His Ala Ser His Thr Gly Ser Trp Ile Gly Leu Arg Asn
     ACC AAG CAT GCC AGC CAC ACC GGC TCC TGG ATT GGC CTT CGG AAC   860

                        230                        235                        240
     Leu Asp Leu Lys Gly Glu Phe Ile Trp Val Asp Gly Ser His Val
     TTG GAC CTG AAG GGA GAG TTT ATC TGG GTG GAT GGG AGC CAT GTG   905

                        245                        250                        255
     Asp Tyr Ser Asn Trp Ala Pro Gly Glu Pro Thr Ser Arg Ser Gln
     GAC TAC AGC AAC TGG GCT CCA GGG GAG CCC ACC AGC CGG AGC CAG   950

                        260                        265                        270
     Gly Glu Asp Cys Val Met Met Arg Gly Ser Gly Arg Trp Asn Asp
     GGC GAG GAC TGC GTG ATG ATG CGG GGC TCC GGT CGC TGG AAC GAC   995

                        275                        280                        285
     Ala Phe Cys Asp Arg Lys Leu Gly Ala Trp Val Cys Asp Arg Leu
     GCC TTC TGC GAC CGT AAG CTG GGC GCC TGG GTG TGC GAC CGG CTG   1040

                        290                        295                        300
     Ala Thr Cys Thr Pro Pro Ala Ser Glu Gly Ser Ala Glu Ser Met
     GCC ACA TGC ACG CCG CCA GCC AGC GAA GGT TCC GCG GAG TCC ATG   1085

                        305                        310                        315
     Gly Pro Asp Ser Arg Pro Asp Pro Asp Gly Arg Leu Pro Thr Pro
     GGA CCT GAT TCA AGA CCA GAC CCT GAC GGC CGC CTG CCC ACC CCC   1130

                        320
     Ser Ala Pro Leu His Ser  *
     TCT GCC CCT CTC CAC TCT TGA GCATGGATACAGCCAGGCCCAGAGCAAGACC   1182

     CTGAAGACCCCCAACCACGGCCTAAAAGCCTCTTTGTGGCTGAAAGGTCCCTGTGACAT   1241

     TTTCTGCCACCCAAACGGAGGCAGCTGACACATCTCCCGCTCCTCTATGGCCCCTGCCT   1300

     TCCCAGGAGTACACCCCAACAGCACCCTCTCCAGATGGGAGTGCCCCCAACAGCACCCT   1359

     CTCCAGATGAGAGTACACCCCAACAGCACCCTCTCCAGATGCAGCCCCATCTCCTCAGC   1418
```

```
ACCCCAGGACCTGAGTATCCCCAGCTCAGGTGGTGAGTCCTCCTGTCCAGCCTGCATCA 1477

ATAAAATGGGGCAGTGATGGCCTCCCAAAAA --------------------------- 1507

----- AAGGAATTC /Sac/Kpn/    /Pst/Sph/Hind/ ━━━━━━━━━━━━━━━━━━
          EcoRI
```

Figure 2

Figure 3: Scheme of pBSF2-L8

```
                                                                    -25
                                          KpnI  Met Asn Ser Phe
                    _____
―――――――――――――――――――――――/Eco/Sac/GGTACC ATG AAC TCC TTC      18

                            -20                  -15                  -10
     Ser Thr Ser Ala Phe Gly Pro Val Ala Phe Ser Leu Gly Leu Leu
     TCC ACA AGC GCC TTC GGT CCA GTT GCC TTC TCC CTG GGG CTG CTC      63

                            -5      Ala Ala Phe Pro Ala  1      Val Pro Pro Gly Glu  6
     Leu Val Leu Pro Ala Ala Phe Pro Ala Pro Val Pro Pro Gly Glu
     CTG GTG TTG CCT GCT GCC TTC CCT GCC CCA GTA CCC CCA GGA GAA     108

                            11                   16                   21
     Asp Ser Lys Asp Val Ala Ala Pro His Arg Gln Pro Leu Thr Ser
     GAT TCC AAA GAT GTA GCC GCC CCA CAC AGA CAG CCA CTC ACC TCT     153

                            26                   31                   36
     Ser Glu Arg Ile Asp Lys Gln Ile Arg Tyr Ile Leu Asp Gly Ile
     TCA GAA CGA ATT GAC AAA CAA ATT CGG TAC ATC CTC GAC GGC ATC     198

                            41                   46                   51
     Ser Ala Leu Arg Lys Glu Thr Cys Asn Lys Ser Asn Met Cys Glu
     TCA GCC CTG AGA AAG GAG ACA TGT AAC AAG AGT AAC ATG TGT GAA     243

                            56                   61                   66
     Ser Ser Lys Glu Ala Leu Ala Glu Asn Asn Leu Asn Leu Pro Lys
     AGC AGC AAA GAG GCA CTG GCA GAA AAC AAC CTG AAC CTT CCA AAG     288

                            71                   76                   81
     Met Ala Glu Lys Asp Gly Cys Phe Gln Ser Gly Phe Asn Glu Glu
     ATG GCT GAA AAA GAT GGA TGC TTC CAA TCT GGA TTC AAT GAG GAG     333

                            86                   91                   96
     Thr Cys Leu Val Lys Ile Ile Thr Gly Leu Leu Glu Phe Glu Val
     ACT TGC CTG GTG AAA ATC ATC ACT GGT CTT TTG GAG TTT GAG GTA     378

                            101                  106                  111
     Tyr Leu Glu Tyr Leu Gln Asn Arg Phe Glu Ser Ser Glu Glu Gln
     TAC CTA GAG TAC CTC CAG AAC AGA TTT GAG AGT AGT GAG GAA CAA     423

                            116                  121                  126
     Ala Arg Ala Val Gln Met Ser Thr Lys Val Leu Ile Gln Phe Leu
     GCC AGA GCT GTG CAG ATG AGT ACA AAA GTC CTG ATC CAG TTC CTG     468

                            131                  136                  141
     Gln Lys Lys Ala Lys Asn Leu Asp Ala Ile Thr Thr Pro Asp Pro
     CAG AAA AAG GCA AAG AAT CTA GAT GCA ATA ACC ACC CCT GAC CCA     513

                            146                  151                  156
     Thr Thr Asn Ala Ser Leu Leu Thr Lys Leu Gln Ala Gln Asn Gln
     ACC ACA AAT GCC AGC CTG CTG ACG AAG CTG CAG GCA CAG AAC CAG     558
```

```
                       161                        166                        171
Trp Leu Gln Asp Met Thr Thr His Leu Ile Leu Arg Ser Phe Lys
TGG CTG CAG GAC ATG ACA ACT CAT CTC ATT CTG CGC AGC TTT AAG   603

                       176                        181
Glu Phe Leu Gln Ser Ser Leu Arg Ala Leu Arg Gln Met
GAG TTC CTG CAG TTC AGC CTG AGG GCT CTT CGG CAA ATG TAGCATG   649

GGCACCTCAGATTGTTGTTGTTAATGGGCATTCCTTCTTCTGGTCAGAAACCTGTCCAC   708

TGGGCACAGAACTTATGTTGTTCTCTATGGAGAACTAAAAGTATGAGCGTTAGGACACT   767

ATTTTAATTATTTTTAATTTATTAATATTTAAATATGTGAAGCTGAGTTAATTTATGTA   826

AGTCATATTTATATTTTAAGAAGTACCACTTGAAACATTTTATGTATTAGTTTTGAAAT   885

AATAATGGAAAGTGGCTATGCAGTTTGAATATCCTTTGTTTCAGAGCCAGATCATTTCT   944

TGGAAAGTGTAGGCTTACCTCAAATAAATGGCTAACTTATACATATTTTTAAAGAAATA   1003

TTTATATTGTATTTATATAATGTATAAATGGTTTTTATACCAATAAATGGCATTTTAAA   1062

AAATTCAGCAAAAAAAAAAAAAAAAAAAAAAGGGATCC/Xba/SaI/Pst/Sph/Hin/   1100
                                       BamHI
```

Figure 4: Scheme of psFc$_\varepsilon$R-1

———————————————————————————— ATTTAGGTGACACTATA

```
                                           2                    7
                                    Met Asn Ser Phe Ser Thr Ser
        EcoRI           KpnI
GAATACACGGAATTCGAGCTCGGTACCCCT ATG AAC TCC TTC TCC ACA ACC    51

                  12                       17                  22
Ala Phe Gly Pro Val Ala Phe Ser Leu Gly Leu Leu Leu Val Leu
GCC TTC GGT CCA GTT GCC TTC TCC CTG GGG CTG CTC CTG GTG TTG    96

              27                       32                  37
Pro Ala Ala Phe Pro Ala Pro Val Pro Pro Gly Glu Asp Trp Gly
CCT GCT GCC TTC CCT GCC CCA GTA CCC CCA GGA GAA GAT TGG GGA    141

          42                       47                  52
Ser Ala Ser Asp Leu Leu Glu Arg Leu Arg Glu Glu Val Thr Lys
TCA GCT TCA GAT TTG CTG GAA AGA CTC CGG GAG GAG GTG ACA AAG    186

                  57                       62                  67
Leu Arg Met Glu Leu Gln Val Ser Ser Gly Phe Val Cys Asn Thr
CTA AGG ATG GAG TTG CAG GTG TCC AGC GGC TTT GTG TGC AAC ACG    231

              72                       77                  82
Cys Pro Glu Lys Trp Ile Asn Phe Gln Arg Lys Cys Tyr Tyr Phe
TGC CCT GAA AAG TGG ATC AAT TTC CAA CGG AAG TGC TAC TAC TTC    276

          87                       92                  97
Gly Lys Gly Thr Lys Gln Trp Val His Ala Arg Tyr Ala Cys Asp
GGC AAG GGC ACC AAG CAG TGG GTC CAC GCC CGG TAT GCC TGT GAC    321

              102                      107                 112
Asp Met Glu Gly Gln Leu Val Ser Ile His Ser Pro Glu Glu Gln
GAC ATG GAA GGG CAG CTG GTC AGC ATC CAC AGC CCG GAG GAG CAG    366

              117                      122                 127
Asp Phe Leu Thr Lys His Ala Ser His Thr Gly Ser Trp Ile Gly
GAC TTC CTG ACC AAG CAT GCC AGC CAC ACC GGC TCC TGG ATT GGC    411

          132                      137                 142
Leu Arg Asn Leu Asp Leu Lys Gly Glu Phe Ile Trp Val Asp Gly
CTT CGG AAC TTG GAC CTG AAG GGA GAG TTT ATC TGG GTG GAT GGG    456

              147                      152                 157
Ser His Val Asp Tyr Ser Asn Trp Ala Pro Gly Glu Pro Thr Ser
AGC CAT GTG GAC TAC AGC AAC TGG GCT CCA GGG GAG CCC ACC AGC    501

              162                      167                 172
Arg Ser Gln Gly Glu Asp Cys Val Met Met Arg Gly Ser Gly Arg
CGG AGC CAG GGC GAG GAC TGC GTG ATG ATG CGG GGC TCC GGT CGC    546

              177                      182                 187
Trp Asn Asp Ala Phe Cys Asp Arg Lys Leu Gly Ala Trp Val Cys
TGG AAC GAC GCC TTC TGC GAC CGT AAG CTG GGC GCC TGG GTG TGC    591
```

```
                            192                 . 197                     202
     Asp Arg Leu Ala Thr Cys Thr Pro Pro Ala Ser Glu Gly Ser Ala
     GAC CGG CTG GCC ACA TGC ACG CCG CCA GCC AGC GAA GGT TCC GCG   636

                            207                   212                     217
     Glu Ser Met Gly Pro Asp Ser Arg Pro Asp Pro Asp Gly Arg Leu
     GAG TCC ATG GGA CCT GAT TCA AGA CCA GAC CCT GAC GGC CGC CTG   681

                            222                   227
     Pro Thr Pro Ser Ala Pro Leu His Ser  *
     CCC ACC CCC TCT GCC CCT CTC CAC TCT TGA GCATGGATACAGCCAGGCC   730

     CAGAGCAAGACCCTGAAGACCCCCAACCACGGCCTAAAAGCCTCTTTGTGGCTGAAAGG   789

     TCCCTGTGACATTTTCTGCCACCCAAACGGAGGCAGCTGACACATCTCCCGCTCCTCTA   848

     TGGCCCCTGCCTTCCCAGGAGTACACCCCAACAGCACCCTCTCCAGATGGGAGTGCCCC   907

     CAACAGCACCCTCTCCAGATGAGAGTACACCCCAACAGCACCCTCTCCAGATGCAGCCC   966

     CATCTCCTCAGCACCCCAGGACCTGAGTATCCCCAGCTCAGGTGGTGAGTCCTCCTGTC   1025

     CAGCCTGCATCAATAAAATGGGGCAGTGATGGCCTCCCAAAAAAAA ------------   1071

     - AAAAGGAATTCGAGCTCGGTACCCGGGGATCCTCTAGAGTCGACCTGCAGGCATGCA

     AGCTTCCGGTCTCCCTATAGTGAGTCCTATTA────────────────────────────
```

# An Eosinophilic Leukemia Line (EoL-3) Expresses Fc$_\varepsilon$R

Figure 5

EP 0 321 842 A1

## Figure 6

Figure 7

Figure 8

πH3M 3.9kbp

πsFcεR 4.7kbp

Table I

Inhibition of IgE-rosette formation of EoL-3
cells with recombinant soluble $Fc_\varepsilon R$

| | | Dilutions | | | |
|---|---|---|---|---|---|
| | | undiluted | 1/3 | 1/9 | 1/27 |
| Exp. 1 | Control | ND | 19.6 % | 20.2 % | 20.1 % |
| | $rFc_\varepsilon R$ | ND | 10.0 | 12.1 | 14.1 |
| Exp. 2 | Control | 22.9 | 20.9 | 19.9 | 18.4 |
| | $rFc_\varepsilon R$ | 10.1 | 12.2 | 14.7 | 16.4 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,P | EP-A-0 286 700 (PROF.T. KISHIMOTO) <br> * Whole document * <br> --- | 1-14 | C 12 N 15/00 <br> C 12 N 5/00 <br> A 61 K 37/02 <br> C 12 P 21/02 |
| Y,D | CELL, vol. 47, 5th December 1986, pages 657-665, New York, US; H. KIKUTANI et al.: "Molecular structure of human lymphocyte receptor for immunoglobulin E" <br> * Whole article, especially figure 4 * <br> --- | 1-14 | |
| Y,D | NATURE, vol. 324, no. 6092, 6th November 1986, pages 73-76; T. HIRANO et al.: "Complementary DNA for a novel human interleukin (BSF-2) that induces B lymphocytes to produce immunoglobulin" <br> * Whole article * <br> ----- | 1-14 | |

TECHNICAL FIELDS
SEARCHED (Int. Cl.4)

C 12 N
C 12 P

The present search report has been drawn up for all claims

| Place of search | Date of completion of the search | Examiner |
|---|---|---|
| THE HAGUE | 28-03-1989 | CUPIDO M. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
........................................................................
& : member of the same patent family, corresponding
    document

EPO FORM 1503 03.82 (P0401)